# EUROPEAN PATENT SPECIFICATION

(11) **EP 1 007 154 B1**
(45) Date of publication and mention of the grant of the patent: **14.01.2004**
(21) Application number: 97947421.0
(22) Date of filing: 07.11.1997
(51) Int. Cl.: A61N 5/06

(54) **INTERVENTIONAL PHOTONIC ENERGY EMITTER SYSTEM**
MEDIZINISCHES EINGRIFFSSYSTEM ZUM EMITTIEREN PHOTONISCHER ENERGIE
SYSTEME EMETTEUR D'ENERGIE PHOTONIQUE INTERVENTIONNEL

(30) Priority: 21.11.1996 US 33335 P; 02.09.1997 US 922263
(43) Date of publication of application: 14.06.2000
(73) Proprietor: Boston Scientific Limited, St. Michael (BB)
(72) Inventor: CROWLEY, Robert, J., Sudbury, MA 01776 (US)
(74) Representative: Greenwood, John David
(86) International application number: PCT/US1997/020435
(87) International publication number: WO 1998/022034

(56) References cited:
- WO-A-92/15253
- WO-A-96/24406
- DE-A- 4 005 743
- US-A- 5 213 569
- US-A- 5 402 792
- VONA D. F. ET AL.: "A test of the hypothesis that cavitation at the focal area of an extracorporeal shock wave lithotripter produces far ultraviolet and soft X-ray emissions" J. ACOUST. SOC. AM. (USA), vol. 98, no. 2, August 1995, pages 706-711, XP002058033
- COLEMAN A. J. ET AL.: "Acoustic emission and sonoluminescence due to cavitation at the beam focus of an electrohydraulic shock wave lithotripter" ULTRASOUND MED. BIOL. (UK), vol. 18, no. 3, 1992, pages 267-281, XP002058034

## Description

### Technical Field

This invention relates to light sources and, more particularly, to miniature light sources for placement inside a body for tissue characterization and treatment.

### Background Information

Physicians have used light for performing diagnosis and therapy of tissue by delivering light to the tissue. Medical applications that use light include, for example, photoactivation of drugs, monitoring of blood glucose levels, tissue spectroscopy, illumination of internal tissue, and tissue ablation. Light system designers have come up with a variety of methods to deliver light to a tissue region of interest. Medium powered light emitting diode arrays placed on catheters and probes are available for activating photoactive drugs such as HPD (Photofrin) and SNeT₂ (Tin Etlopurpurin Dichloride) to perform photodynamic therapy (PDT). Most of the light system that generate high energy light are external light sources that use optical fibers to deliver the light to a variety of anatomical locations inside the body. Other ways to deliver energy to an internal tissue region include direct heating via conduction loss through a catheter or balloon electrodes, radioactive seeding passed through needles or catheters, inserting cryogenically cooled catheter tips, and using various light diffusers or ultrasonic transducers.

In general, health services under managed care guidelines require that medical procedures be more effective, faster, and inexpensive. Several promising medical diagnosis and treatment systems using light wave energy have failed to become commercially successful due to the high cost of the instruments. Most high energy light systems are expensive, large, and complex because they require an external light source, light conducting fibers, transducers, and connectors.

The use of optical fibers to deliver light presents several problems. In order to transport an adequate amount of light energy from the light source to an internal tissue region, a significant amount of optical fibers must be included in an interventional device. An interventional device (e.g., catheter, endoscope, guide wire, needle or introducer), however, does not include a lot of space and higher quality optical fibers, which take up less space, are expensive. Optical fibers also lack mechanical properties necessary to be used with an interventional device. Optical fibers can break when flexed and have a relatively high stiffness as compared to conventional catheter materials. Therefore, it is difficult to design a flexible tip for a catheter that includes optical fibers. Overall flexibility of an interventional device that includes optical fibers is limited. Furthermore, optical fibers require an expensive terminating connector and must be properly coupled to afford adequate light throughput. Signal efficiency of fiber based devices depends greatly upon the ability of the device to couple sufficient light into the fibers at the desired wavelength, but it is a challenge to efficiently couple light from a lamp source into fibers with small diameters.

Known high energy light systems also tend to cause undesirable side effects. The high intensity light, which is necessary for medical procedures, can cause thermal destruction of normal tissue regions, since the light signals have high intensity as well as long duration.

WO-A-92/15253 is prior art to the present application. WO-A-91/15253 discloses an interventional device comprising a housing, an acoustic transducer and an acoustic conducting medium inside the housing and adjacent the acoustic transducer. The device has a lens for focussing ultrasound in the tissue outside the housing. The present invention is as claimed in the independent claims optional features are recited in the dependent claims.

The present invention relates to improved high energy light systems for use in interventional devices for medical applications. The high energy light systems according to the invention include miniature light sources capable of generating high intensity modular light waves and capable of being placed at or near the tips of various interventional devices (e.g., a catheter, endoscope, guide wire, needle or introducer). The present invention, therefore, eliminates the need for expensive proximally located light sources, transducers, fibers, and connectors. The present invention further provides light sources that generate modular light output in a spectrum ranging from the ultraviolet to x-rays. The high output, short duration light waves allow safe operation without excessive heating effects.

In general, in one aspect, the invention features an interventional device including a miniature light device for generating and delivering high energy modular photonic energy to an internal tissue region for diagnostic and/or therapeutic purposes. The light device is capable of being placed at or near a distal end of the interventional device, eliminating the need for light carrying conduits to deliver light generated by an external light source. The device may further include a feedback system and a light guide for supplying light output to the feedback system.

The light device is a sonoluminescent light module. The sonoluminescent light module includes a housing, an acoustic transducer and an acoustic conducting medium. The acoustic conducting medium is positioned inside the housing adjacent the acoustic transducer. The acoustic transducer comprises a piezoelectric material and a wave matching layer. The sonoluminescent light module is capable of generating light spectrum in the X-ray region.

The foregoing and other objects, aspects, features, and advantages of the invention will become more apparent from the following description and from the claims.

### Brief Description of the Drawings

In the drawings, like reference characters generally refer to the same parts throughout the different views. Also, the drawings are not necessarily to scale, emphasis instead generally being placed upon illustrating the principles of the invention.
FIG. 1 is a schematic representation of a medical apparatus comprising an energy source, a signal conduit, and an energy emitter inserted in an interventional device.
FIG. 2A is a diagram of an arc lamp for placement inside a body.
FIG. 2B is a diagram of an arc lamp in communication with a feedback system.
FIG. 3A is a diagram of a discharge tube placed at a distal end of an interventional device.
FIG. 3B is a diagram of a discharge tube in communication with a battery and a capacitor.
FIG. 3C is a diagram of the discharge tube of FIG. 3A placed inside a balloon catheter deploying a stent.
FIG. 4 is a diagram of an embodiment of the invention of a sonoluminescent light source in communication with a pulse generator.
FIG. 5A is a diagram of a multi-electrode spark gap for placement inside an interventional device.
FIG. 5B is a diagram of an incandescent light source.
FIG. 6 is a diagram of a laser diode driven light source placed at a distal end of an interventional device.
FIG. 7A is a diagram of a discharge lamp in communication with a transformer.
FIG. 7B is a diagram of a fluorescent lighting device driven by a Gunn diode.
FIG. 8A is a diagram of a catheter for use with a miniature light device.
FIG. 8B is a diagram of a needle for use with a miniature light device.
FIG. 8C is a diagram of a guide wire for use with a miniature light device.
FIGS. 8D and 8E are diagrams of endoscopes for use with a miniature light device.

### Description

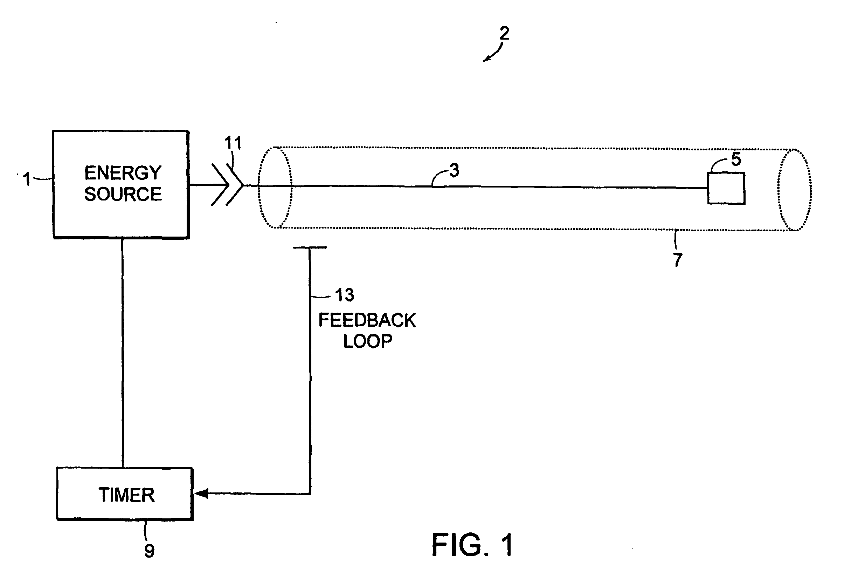

Referring to FIG. 1, a medical apparatus 2 includes an energy source 1, a signal conduit 3, a light device 5 and an interventional device 7 for introducing the light device 5 to an internal tissue region. The energy source, for example, may be an external battery, power supply, pulse generator or RF generator. The energy source 1 may be controlled by a computer or microprocessor. The energy source 1 delivers energy to a port 11. The medical apparatus also includes a timer 9 for limiting the duration of the energy delivered to the port 11. A signal conduit 3 extends along the length of the interventional device 7 and provides electrical communication between the port 11 and the light device 5. The signal conduit 3, for example, comprises an ordinary twisted wire, coaxial cable, metallized traces or other suitable signal carrying conduit. The signal conduit 3, in bipolar mode, includes at least one signal wire and one return wire. Alternatively, the signal conduit 3 may operate in monopolar mode in certain instances. In one embodiment, where the interventional device 7 is a catheter, the signal conduit 3 is a wire, having a diameter ranging anywhere from about 0.005 inches to about 0.080 inches. A wire having a diameter in this range does not greatly impact the flexibility or size of the catheter 7. In another embodiment, metal bodied catheters, guide wires or needles provide the return path using the conductivity of the metal body.

Still referring to FIG. 1, the medical apparatus 2 further includes a feedback loop conduit 13. The feedback loop conduit collects and returns a small portion of the light energy generated by the light device 5. In one embodiment, where the interventional device 7 is an optically transparent catheter, the catheter body conducts some of the light generated at a distal tip of the catheter 7 back to a proximal end of the catheter 7, permitting an operator to observe or confirm the operation of the medical apparatus 2 simply by looking at the proximal end of the catheter 7. In another embodiment, the returned energy is coupled to the energy source 1 to provide a controlling feedback in response to onset of illumination, intensity or spectral components.

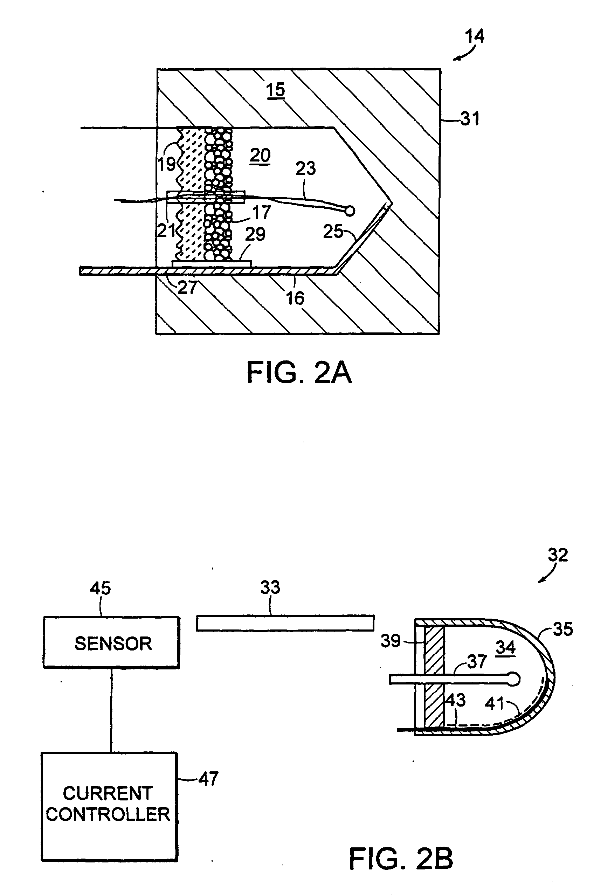

Referring to FIG. 2A, the light device, not belonging to the invention, is an arc lamp 14. An arc lamp is an electric lamp in which light is produced by an arc made when current flows through ionized gas between two electrodes. The arc lamp 14 includes a pair of electrodes 23, 27. The electrodes 23, 27 are spaced from one another for striking an arc. A housing 15 surrounds the arc lamp 14. In one embodiment, the housing comprises quartz 15. The electrodes 23, 27 are sealed inside a cavity 20 defined by the housing 15. A sintered metal 17 with a glass or epoxy 19 seal the arc lamp 14. In one embodiment, the sintered metal 17 is copper. The sintered metal 17 absorbs some of the vapors generated from the operation of the arc lamp 14 and reduces clouding during operation. The glass or epoxy seal 19 yields at high pressure, preventing the housing 15 from fracturing. Sealing the arc lamp 14 prevents any debris or escaping gas generated by the arc lamp 14 from leaving the confines of the interventional device 7. An insulator 21 isolates the first electrode 23 from the sealing material 17, 19. In one embodiment, a distal end of the first electrode 23 has a hemispherical shape and comprises coating of a material such as carbon or tungsten. The second electrode 27 forms the return electrode. The second electrode 27 is formed along one side 16 of the housing 15. A separator 29 protects the second electrode 27 and prevents current from the second electrode 27 from flowing through the sintered metal 17. The inner surface of the housing 15 is coated with a conductive trace of aluminum 25. In one embodiment, a vacuum metallizing process coats the inner surface. The thickness of the aluminum is enough to sustain an arc for a short period of time, while transmitting a useful amount of light energy.

Still referring to FIG. 2A, the shape of the housing 15 permits collecting and redirecting some of the light energy generated by the arc lamp 14 to the proximal end of an interventional device in communication with the arc lamp 14. The flat front surface 31 of the housing 15 may include additional lenses. Alternatively, masking may modify the shape of the housing 15. In addition, the flat surface 31 may be coated with a reflective substance. In this embodiment, the thick wall of the housing 15 may be coupled to one or more light guides, comprising optical fibers, transparent cylindrical catheter bodies and the like, to conduct some of the reflected light energy to the proximal end of the interventional device for the purpose of providing a feedback system.

In another example, the housing 14 provides means for cooling the arc lamp 14. The housing may include passages for a cooling fluid to flow. Alternatively, a jacket of water, air or other fluid may surround the housing 14.

Referring to Figure 2B, a light system 32, not belonging to the invention, having an arc lamp 34, illustrates the usefulness of a feedback mechanism. To "strike an arc," one must either bring two electrodes close together and then separate them by a distance, or if that is not possible, raise the voltage applied to the electrodes gradually until an arc is struck and then, drop the voltage quickly. An ordinary current sensing circuitry may assist this process by sensing an increase in current flow as the arc is struck and sustained. While the current sensing circuitry may be useful, a better arrangement is to sample the actual light output, which is responsive to slight changes in applied power and erosion of the electrodes within. This capability is especially valuable, since the burning time of the arc may be in the order of a few milliseconds, short enough to prevent heating and destruction of the carrying member or the surrounding anatomy.

Still referring to Figure 2B, the light system 32, not belonging to the invention, includes an arc lamp 34 in communication with a feedback system 36. The arc lamp 34 comprises a first metal electrode 37 disposed at the center of a cavity defined by a hemispherical quartz dome 35. The electrode 37 is held stationary by copper wool 39, tightly packed to seal the arc lamp 34. The copper wool 39 absorbs the fumes emitted by the arc. The light system 32 includes a second electrode 43 or a return path for the arc. A flash metallization 43 process may form the return path 43. The return path 43 formed by flash metallization is sufficient for a short duration emission of an extremely bright output of wide spectral bandwidth including infrared (IR), visible and ultraviolet (UV) components. In one embodiment, the dome surface has a layer 41 of mercury, which enhances certain spectral wavelengths. The feedback system 36 includes a light guide 33, which is in communication with the arc lamp 34 at a distal end and a sensor 45 at a proximal end. The sensor 45 supplies light output to the current controller 47.

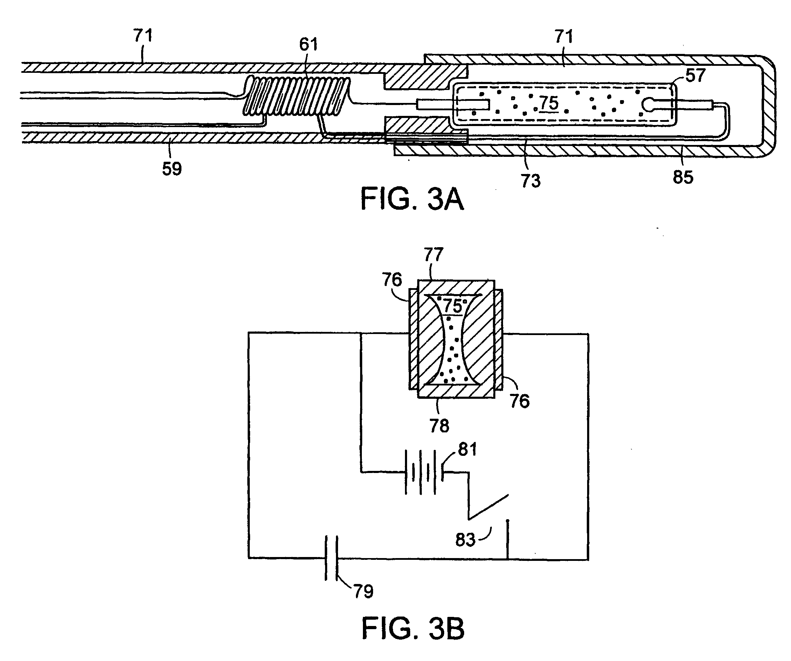

Referring to Figure 3A, the light device is a discharge lamp 57, which does not belong to the invention. In a discharge lamp, light is produced by an electric discharge between electrodes in a gas. A rod-like assembly 59 has a discharge tube 57 mounted on a distal end. The assembly 59 permits placement of the discharge tube 57 through various lumens including, for example, the lumen of a catheter with an optically clear structure or an endoscope. A transformer 61 disposed proximal to the discharge tube 57 provides a voltage step. The transformer 61, for example, consists of a copper wire wound around a cylindrical form and tapped at various points along the length of the wire. A light system that generates short duration light waves permits the use of wire gages that would otherwise be too small to withstand heating effects in continuous service. The transformer 61 may use a wire as small as 0.0127 cm (0.005 inches) in diameter, as long as it is capable of supplying adequate current at relatively high voltage. An example of a suitable wire is a common enamel-covered copper wire. The turns ratio between the transformer primary and the transformer secondary coil determines the step up ratio of the transformer 61. The transformer 61 requires only one tapped coil, thus saving space. The coil may be wound around a metallic core such as iron, which improves its efficiency. The core diameter ranges from about 0.0102 cm (0.004 inches) to about 0.2 cm (0.080 inches). The core length ranges from about 0.236 cm (0.093 inches) to about 2.54 cm (1.0 inch) or even longer. In one embodiment, one or more layers of copper wire wraps around flexible cores of thin strips of iron to provide a flexible assembly that does not greatly interfere with the flexible characteristics of an interventional device.

Still referring to FIG. 3B, the discharge tube 57 includes a third wire or capacitively coupled electrode 71 placed adjacent the discharge tube 57. The third wire 71 extends along an interventional device and communicates with the reference ground of a power discharge source. The third wire 71 improves the flash output of the discharge tube 57 by providing an approximately equipotential charge along the length of the discharge tube 57, thereby improving reliability, while reducing the peak voltage needed for flash onset.

In one example, the discharge tube 57 is mounted directly at the distal end of the transformer 61. In another embodiment, the discharge tube 57 is separated from the transformer 61 with wires 73. A variety of materials can fill the discharge tube 57. In one embodiment, the discharge tube is a flash tube filled with gas 75 such as xenon, argon or krypton, providing various spectral output. In another example, combinations of gases with other substances, such as xenon and a chloride fill the flash tube 57. The combination of xenon and chloride produces output with prominent spectral lines in the ultraviolet (UV) region at around 308 nm or shorter. It is difficult to deliver spectral output in the UV region through an ordinary optical fiber due to loss through attenuation. In one example, the flash tube 57 is frosted or coated with a phosphorescent or fluorescing material such as borax.

An interventional device, not forming part of the invention, may include an elongated discharge tube 77 or a series of tubes capable of being passed though a channel of an interventional device. The length of the intervention device, for example, may be 2 meters or more. The discharge tube 77 diameter, for example, may be approximately 0.317 cm (0.125 inches) and the discharge tube 77 length, for example, may be approximately 2.54 cm (1 inch). In the example of FIG. 3A, a transparent sheath housing 85 surrounds the discharge tube 77 to protect the discharge tube 77 and to prevent the discharge tube 77 from contacting the wall of a bodily channel being illuminated. Examples of suitable housing 85 materials include polyethylene and polypropylene. UV absorption, however, can be high in such materials. Therefore, where generation of UV light is desired, these materials must be sufficiently thin walled (eg., about 0.005 cm (0.002 inches)) to reduce the net loss of UV energy to a manageable level. The relative lack of rigidity of the sheath material can be compensated by stretching the material, which has been formed into a cylindrical sheath over the discharge tube 77 or by inflating a section of the discharge tube 77 so that it takes on a more rigid form.

Referring to FIG. 3B, an equipotential discharge tube 77 reduces the possibility of hot spot formation. An equipotential discharge tube 77 has a shape, which provides a more even voltage gradient across a section of the tube 77, and therefore a more distributed discharge upon firing. A dielectric material 78 surrounds the tube 77. A pair of electrodes 75, 76 formed by metallization contacts opposite sides of the dielectric material 78. A suitable dielectric material 78, for example, includes glass or polystyrene. In one example, the voltage gradient across the electrodes 76 is made more uniform by reducing edge effects. Edge effects may be reduced by creating a local condition with greater amount of gas 75 in the tube and a smaller amount of the dielectric material 78. In one example, the discharge tube 77 is in communication with a current source 81 such as a battery and a capacitor discharge storage 79. The battery 81 charges the capacitor 79 to store energy, and the stored energy is discharged and applied to the discharge tube 77 by opening a normally closed switch 83. In another example, an RF current of either a continuous waveform (CW) or a pulsed form effectively creates a high voltage at the electrodes 75, 76 of the discharge tube 77 to generate light output.

In one example, an endoscope provides means for introducing the light devices of Figures 3A and 3B. Once the light device is properly positioned, the light device discharges a desired spectrum of light to perform a medical procedure. One medical procedure performed in this manner is destroying or ablating a thin layer of cells on the surface of an organ or the inner wall of a vessel such as the esophagus using ultraviolet energy. The broad spectrum of emission of a xenon type flash tube, which contains IR, visible and UV components, for example, may be used to ionize, heat and/or irradiate a surface. This action may also kill foreign cells such as bacteria or various viruses. This procedure is described in a commonly-owned U.S. provisional patent application, namely U.S. provisional patent application serial no. 60/033,333 published as EP-A-939 894. Another commonly-owned U.S. provisional patent application is U.S. provisional patent application serial no. 60/033,334. Another medical procedure performed with such a device is tissue spectroscopy. Short duration UV pulses can excite intrinsic fluorophores that may be present in a tissue region. For this application, the output of the discharge lamp may be filtered so that its radiation is restricted to the blue or UV region of the spectrum. Still another medical procedure performed, includes activation of a photoactive drug.

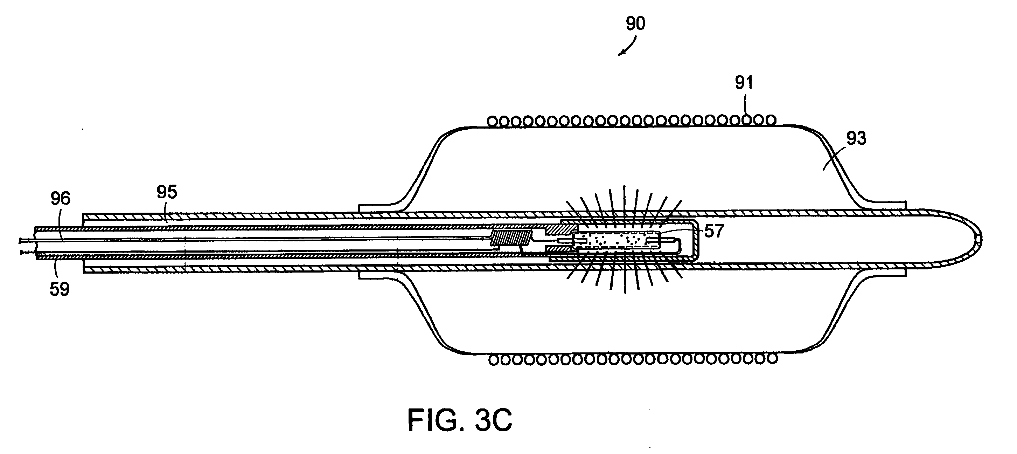

Referring to FIG. 3C, a balloon catheter 90 includes the light device of FIG. 3A. Air or fluid may inflate the balloon portion 92 of the catheter 90. The balloon 92 includes a polymeric stent 92. The discharge lamp 57 inside the balloon 93 hardens the distended polymeric stent 92 by irradiating the polymeric stent 91. In one example, the polymeric stent 91 comprises a UV-curable epoxy or adhesive to assist in hardening of the stent 91. Loctite 3761® adhesive "Litetak"® is an example of a UV-curable adhesive. A fibrous stent may be hardened by impregnating the stent with some of the UV-curable adhesive and illuminating it with the intense light output of the discharge tube in-vivo. The inflation lumen 95 may include a cooling fluid to cool the discharge tube 57. An inner sliding member 96 may be used to adjust the position of the discharge tube 57 inside the balloon 93.

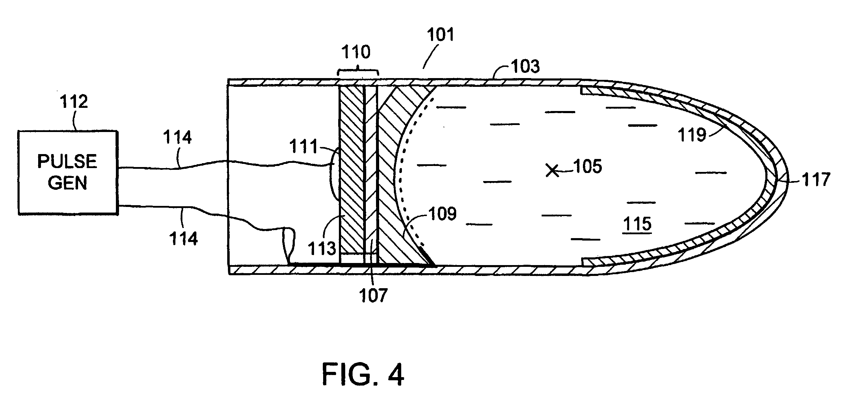

All of the light devices described thus far generate photonic energy in the infrared (IR), visible and ultraviolet range of the spectrum. Referring to FIG. 4, a sonoluminescent light device according to the invention 101 provides light output in the X-ray spectrum region. The term "sonoluminescence" refers to luminescence produced by high frequency sound waves. The operation of the sonoluminescent effect is currently somewhat of a mystery; however, it has been shown that sufficiently high acoustic powers may be generated with practical ultrasonic transducers and the sound waves focused to a point in a sound conducting medium may emit a short pulse of light energy including the ultraviolet region to X-ray region. Therefore, it becomes possible to generate X-rays at the distal tip of a flexible catheter.

Still referring to FIG. 4, the sonoluminescent light device 101 includes a housing 103, an acoustic transducer 110, and an acoustic conducting medium 105. The housing 103 encloses the acoustic conducting medium 105. The acoustic conducting medium 105 is disposed in the pathway of sound waves generated by the acoustic transducer 110. The acoustic transducer 110 includes a piezoelectric material 113 and an integral matching layer 107. Lead zirconate-titanate is an example of a piezoelectric material 113, which can be used to form the acoustic transducer 110. Other suitable piezoelectric materials 113 may also be used to convert electrical energy to mechanical energy.

The sonoluminescent light device 101 further comprises a focusing lens 109, which is curved to provide a sharp spot of focused sound waves in the acoustic conducting medium 105. The focusing lens 109 sits in between the acoustic transducer 110 and the acoustic conducting medium 105. The sonoluminescent light device 101 comprises two electrodes. The first electrode 111 attaches to the back of the piezoelectric material 113 . The second electrode attaches to the face of the acoustic transducer 110. The thickness of the piezoelectric layer 113 determines the frequency of the operation. In one embodiment, the wave matching layer 107 is a 1/4 wave matching layer 107 made of a material such as silver filled epoxy. The wave matching layer 107 serves as both an electrode and a matching layer. In another embodiment, the wave matching layer 107 is shaped into a focusing lens to concentrate the ultrasound beam. In one embodiment, the acoustic conducting medium 115 comprises water. In another embodiment, the acoustic conducting medium 115 comprises a solid substance or target, on which the sonoluminescent effect can be observed. A pulse generator 112 provides a high voltage pulse or pulses to the transducer 110 via cable lines 114. A train of pulses may be employed to produce a series of light or X-ray output events, and the pulses may be stepped up or down in voltage using a transformer.

Still referring to FIG. 4, in one embodiment, the distal end 117 of the housing 103 is shaped to permit further reflection and concentration of the acoustic waves. The acoustic signals, when insonified, may radiate photonic energy including X-rays.

The sonoluminescent light 101 can be implanted inside a body. The sonoluminescent light device 101 can also be inserted inside an interventional device and the focal point of the acoustic signals lies outside the light assembly and inside the interventional device. The interventional device may simply be a cap, cover, or needle. Alternatively, the sonoluminescent light may be placed within a catheter, guide wire, endoscope or introducer.

The sonoluminescent phenomenon is currently under investigation and may affect matter and living tissue in previously unobserved ways and the use of a medical device in conjunction with a transducer capable of generating the sonoluminescence may find uses that have not been anticipated.

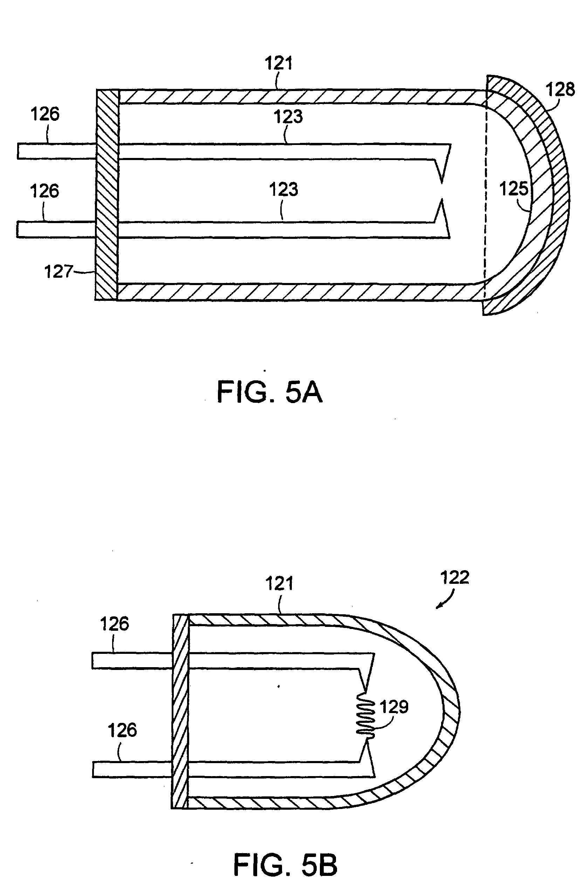

Referring to FIG. 5A, a multi-electrode spark gap module 121, which does not form part of the invention, includes a pair of electrodes 123 held inside a housing 121. The multi-electrode spark gap module 21 is capable of generating a spark across the gap of the electrodes 123, in response to the application of electronic pulses. A spark is a short duration electric discharge caused by sudden breakdown of air or some other dielectric material separating two terminals. The electric discharge produces a flash of light. The spark gap module 121 operates in a way similar to the arc lamp of FIG. 2A, except that the cap does not necessarily have to be conductive in order to generate an emission when the current flow is established at the onset of discharge. The spark gap module 121 provides certain advantages, such as lower heat generation during operation and ease of manufacturing.

Still referring to FIG. 5A, the spark gap module 121 includes electrodes 123 sealed in a glass envelope 125, in an manner similar to conventional spark gaps used to control static discharge. Leads 126 supply electrical power to the electrodes 123. An insulator 127 seals the module 121 and separates the leads 126 from each other. Examples of materials appropriate to form the insulator 127 include plastic, glass and other nonconductive materials.

It is possible to use the light energy of this relatively small electrical discharge produced by a spark gap module 121 to excite a volume of nearby tissue and determine its colors and fluorescence. The spectrum of the light generated by a spark gap module 121 contains blue and UV portions of the spectrum. This range is particularly useful for exciting fluorophors which may be present in the tissue. A filter layer 128 disposed at the distal end of the spark gap module 121 enhances the output of the blue and UV region of the spectrum. The filter layer 128 may comprise an inexpensive dyed plastic dip coat or a more expensive dichroic coating.

Referring to FIG. 5B, an incandescent light source 122, which does not form part of the invention, provides high intensity short duration light output. The incandescent light source 122 has the same basic structure as the spark gap module of FIG. 5A, except that the incandescent light source 122 includes a filament 129 (i.e., non-gap). In one example, the incandescent lamp 122 includes two electrodes encased in a housing 121 and a tungsten filament 129 bridging the two electrodes. The incandescent light source 122 generates high intensity, short duration light without generating excessive heat. The incandescent light source 122 can generate emissions of less than 100 milliseconds with color temperature of about 5000 degrees Kelvin, which includes substantially blue and UV light energy. Other filaments, vacuum or gas-filled enclosures, including oxygen filled and oxidizing filaments like those used in flash bulbs may be used to generate light of various colors.

Certain medical procedures such as photodynamic therapy (PDT) or tissue spectroscopy including fluorescence and Raman spectroscopy require monochromatic light output at high intensities. One method of generating a monochromatic output is to use a filter. This method, however, may be inefficient when highly attenuative filtering techniques are employed. Another method of generating a monochromatic light is to use a laser. Typical laser diodes, which are commonly found in laser pen pointers have outputs in the red region with power levels typically in the 1-7 mW range. Lasers can be made very small in size using semiconductor fabrication processes. A typical laser diode assembly is about 0.952 cm (0.375 inches) in diameter, but most of that size is attributed to the case and tabs for solder connections. The actual light generating portion of the diode is in the order of a few microns in thickness and a few tens of microns in width and length. Therefore it is reasonable to predict that laser diode fabrication in the range of 0.0254 cm (0.010 inches) to 0.203 cm (0.080 inches) will be practical and economical for use in catheter based devices. One drawback of the laser diode is that it is available in only a few wavelengths mostly within the IR and red regions, and none currently in the UV regions. Advances in semiconductor processing and laser diode physics portend that UV laser diodes will exist in the future, but in the meantime a practical way to double the frequency of operation is by introducing a volume of an optically nonlinear material followed by a filter that doubles the frequency of the laser diode.

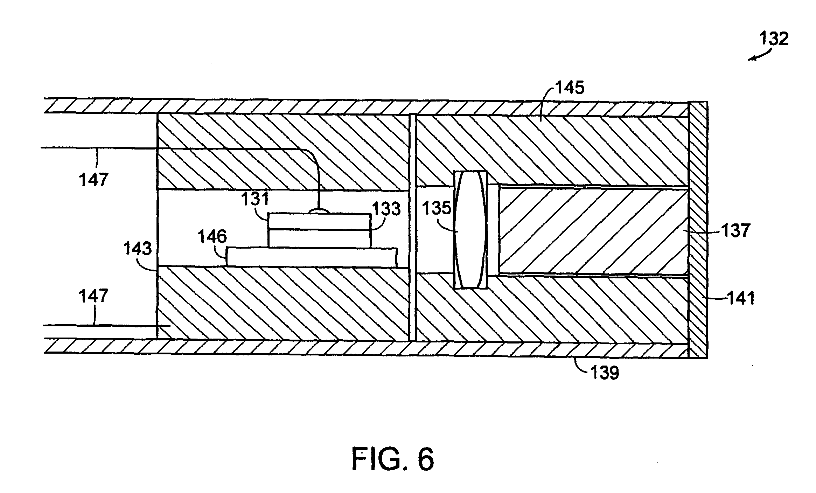

Referring to FiG. 6, the light device 132, which does not form part of the invention, includes a laser diode 131 and a frequency multiplier 137. The laser diode 131 is capable of producing coherent laser light, which emanates from a narrow gap 133 in the diode structure 131. The frequency multiplier is a KDP crystal 137 placed distal to the laser diode 131, and the laser diode 131 and the frequency multiplier 137 are held in proximity to the tip of a carrying body 139. A high pass filter 141 is mounted on the opposite side of the laser diode 131 so that any light of the fundamental frequency is absorbed or reflected and only the multiplied waveform remains. A diode mount 143 and a lens holder 145 maintain the relationship of the elements within the carrying body 139. A pair of wires 147 supply DC power to the diode 131 from a connector at the proximal end of the carrying body 139. A heat sink 146 supporting the laser diode 131 prevents overheating of the laser diode 131. In one example, the laser diode 131 is cooled further by fluid flow in the interventional device. It should be pointed out that the efficiency of frequency doubling in this manner is extremely poor and that outputs greater than -40dB relative to the source is expected. In the past, this feature has made most applications of frequency multiplying crystals to laser diode devices impractical. In the present example, however, the efficiency is tolerable since the light loss functions of intervening materials may be negligible and the power requirements are low. In another example, the light device 132 includes an ordinary light emitting diodes, which generates a non-coherent output.

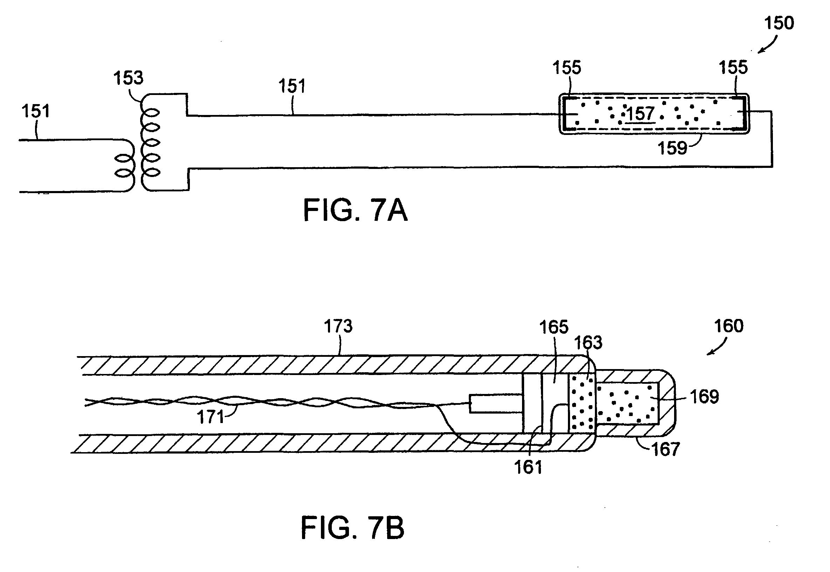

A fluorescent lighting device can generate monochromatic or relatively narrow band light wave energy. The fluorescent lighting device may be gas filled tubes, which fluoresce at known wavelengths and produce output spectra composed of discrete lines. Referring to FIG. 7A, an RF driven fluorescent lighting device 150, which does not belong to the invention, generates monochromatric light. The fluorescent light device 150 may be inserted inside an interventional device such as a catheter or a guide wire. The fluorescent light device 150 includes a tube 159 filled with argon gas 157. The tube 159 is pressurized or partially evacuated and then sealed. A pair of signal wires 151 electrically connect a transformer 153 to the tube 159 through a pair of electrodes 155 located on opposite sides of the tube 159. The transformer 153 may step up an ordinary 60 Hz AC voltage to a higher voltage. Higher frequencies ranging from about 60Hz to about 200GHz provide greater efficiencies and more stable light output. In one example, an RF generator connects to a proximal end of an interventional device carrying the lighting device 150. This example provides even greater efficiencies, since there is no need to supply RF energy to the interventional device via external means.

Referring to FIG. 7B, a fluorescent light device 160, not belonging to the invention, includes a Gunn-effect diode 161 placed adjacent a resonant dielectric resonator 163. Materials suitable to form the resonator 163, for example, include yttrium-iron-garnet (YIG) and other high dielectric materials. These materials provide useful tuning ability and high efficiency oscillation, which results from the diode 161 being used as a relaxation oscillator. In the example of FIG. 7B, the light device 160 includes an additional cavity resonator 165 to provide a higher RF voltage to a gas tube 167. In one example, the cavity resonator 165 is filled with glass microspheres to improve strength of the assembly, which would otherwise be hollow. The gas 169 placed inside the gas tube 167 may be any gaseous substance that will not explode when excited with RF energy, will fluoresce when DC current is applied through wires 171 in electrical communication with the diode 161 and resonator 165, causing the diode 161 to emit RF energy.

Examples of other types of light generating systems not specifically described herein are electroluminescent panels, mechanical sparking, various incandescent and combustion generated light, chemical luminescence and others. Numerous light sources can be placed at a distal end of an interventional device by a combination of miniaturization and use of short duration energy.

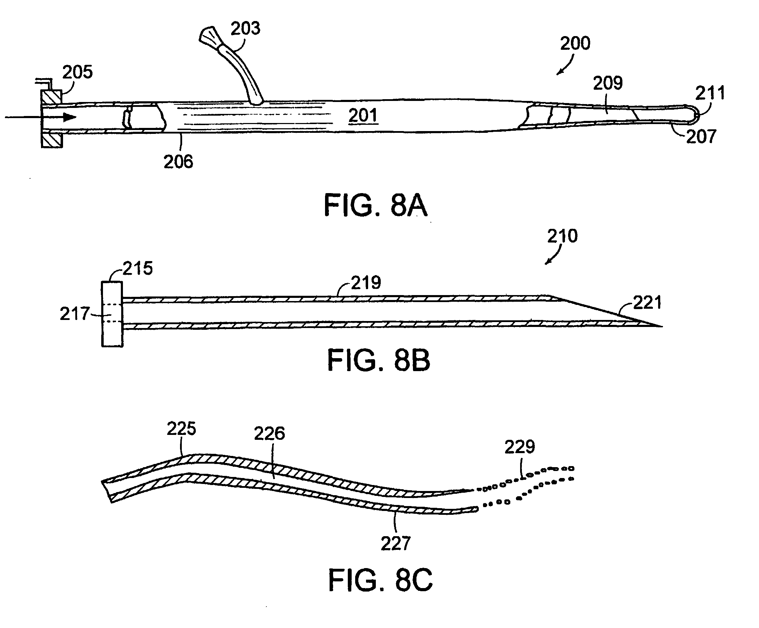

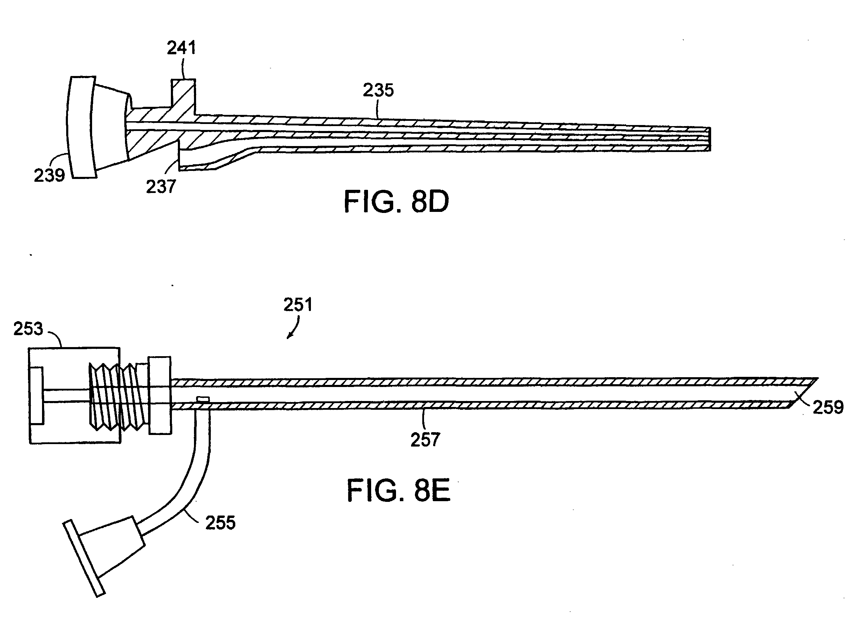

Referring to FIGS. 8A, 8B, 8C, 8D, and 8E, an interventional device having at least a portion that is optically transmissive may include any of the aforementioned energy sources. The interventional device is sized to permit passage through at least one of its lumens. A portion of the interventional device may function as the housing for the various light devices. It should be understood that any of the features herein referred to as catheter or catheter sheath also refers to other interventional devices such as a guide wire, guiding catheter, endoscope and needle art.

Referring to FIG. 8A, a catheter 200 may be used to introduce a light device inside a body. The catheter 200 includes a catheter body 201, a fluid port 203, an optically transparent window 209 and an aperture 211 located at a distal end 207 of the catheter 200. The catheter body 201, for example, comprises an extruded plastic such as polyethylene, nylon, PET, or polyimide. The fluid port 203 located near a proximal end 206 of the catheter 200 allows introduction of various substances such as drugs, fluorescing agents, ultrasound transducers, pressure transducers, flush, cooling or irrigation fluids or optical coupling fluids. The fluid port 203 comprises a side arm and a Luer fitting, as commonly used in the catheter art. The proximal end 206 of the catheter 200 further comprises a connector 205 capable of electrical and/or optical connection. In the case of a closed loop feedback system as shown in FIG. 2B, the catheter 200 includes both optical and electrical connection made simultaneously. The distal end 207 of the catheter 200 is fitted with an optically transparent window 209 and an aperture 211, through which fluid or light wave energy may pass. The material for the transparent window 209 is chosen to efficiently transmit various emission wavelengths generated by the light system incorporated inside the catheter 200. For example, where the light device generates UV light, compromise between strength of the window 209 and the thickness of the window 209 may be necessary, since UV spectrum tends to attenuate when passing through a plastic material. In the embodiment of FIG. 8A, an aperture located adjacent the light source allows the generated light to reach an interior tissue region. In another example, a low loss glass such as quartz is bonded to the distal tip 207 of the catheter 200. In the embodiment of the X-ray generating sonoluminescent light source of Figure 4, the X-ray radiation may be emitted through a denser material such as a metal, which may serve to house and shield, redirect or focus the X-rays at the distal tip 207 of the catheter 200. In still another example, the energy producing transducer protrudes through an open end of the catheter 200.

Still referring to FIG. 8A, in one example, a light device placed inside the catheter 200 contacts the inner surface of the distal end 207 of the catheter 200 to affix the light device location. In another example, the light device is located in registration with a window 209 or another location throughout the length of the catheter 200. The placement of the light device is adjustable using a proximal holder, which allows the user to pre-position the light device inside the catheter 200 or to move the light device and the catheter 200 relative to each other during use. In one example, moving the light device relative to the catheter 200 during use allows the light device to illuminate various regions of the anatomy without having to move the catheter. In another example, the light device inside the catheter 200 creates an image either by rotating the catheter 200 or the light device inside the catheter 200, or by sliding the light device relative to the catheter body 201 to effect a scanning action.

Referring to FIG. 8B, a hollow needle 210 is configured to introduce a light device of the present invention near an interior tissue region. The needle 200 includes a shaft 219, a beveled tip 221 at a distal end of the needle 210 and an aperture 217 sized to accept the light device at a proximal end of the needle 210. In one example, the needle shaft 219 comprises stainless steel with a wall thickness of about 0.00762 cm (0.003 inches) and a length of about 210 millimeters. The beveled tip 221 may be sharpened to permit easy insertion of the needle 210 and a pathway for the light device.

An operator uses an external x-ray or ultrasound imaging technique to first locate an internal tissue region of interest. The operator then inserts the needle 210 inside the body under image guidance until the tip 221 reaches the region of interest. The needle 210 allows the light device to be inserted into the body through the aperture 217 of the needle 210 and be located near the tissue region. The operator may confirm the position of the light device using the aforementioned image guidance. Once satisfied that the light device is in the proper place, the operator applies power to the light device to generate light.

Referring to FIG. 8C, a guide wire 225 placed inside a preexisting channel may deliver a light device of the present invention near an internal tissue region. The guide wire 225 has a single central lumen 226 extending through the guide wire body 227 and a guide wire coiled tip 229. The guide wire body 227, for example, comprises hypo tube, plastic extrusion, or wound wires. The tip 229, for example, comprises coiled stainless steel or platinum wires. The outside diameter of the guide wire 225 ranges from about 0.089 cm (0.035 inches) to about 0.0254 cm (0.010 inches.) The inside diameter of the lumen 226 ranges from about 0.0812 cm (0.032 inches) to about 0.0102 cm (0.004 inches). The guide wire 225 length ranges from about 100 cm to about 200 cm. The guide wire 225 with a length in this range is useful for reaching the GI tract, brain, heart and other remote internal tissues. In another embodiment, a shorter guide wire 225 introduces a light device to less remote areas such as the tear ducts or breast milk ducts.

Referring to FIG. 8D, an endoscope 235 may deliver a light device of the present invention near an internal tissue region through an access port 237. The endoscope 235 further includes an eye piece 239 and a flush lumen 241 used to flush a guide the location of the tip of the endoscope. The access port 237 may be relatively large to allow a light device to easily pass through. In one embodiment, the access port 237 has a diameter up to about 0.475 cm (0.187 inches). The endoscope 235 is useful for delivering high energy emission such as that afforded by the xenon flash tube module to an area of the body such as the esophagus.

Referring to FIG. 8E, an introducer 251 may deliver a light device of the present invention near an internal tissue region. The introducer 251 includes a Touhey-Borst fitting 253, a side arm 255, a single lumen plastic sheath 257, and an open distal end. In one embodiment, the open distal end 259 of the introducer 251 is inserted into arteries or veins and another interventional device comprising a light device is introduced into the internal tissue region through the introducer 251. The guide wire shown in FIG. 8C, the catheter shown in Figure 8A and the needle shown in FIG. 8B are examples of interventional devices, which may comprise a light device of the present invention and which may pass through the introducer 251 to be placed near an internal tissue region of interest.

Variations, modifications, and other implementations of what is described herein will occur to those of ordinary skill in the art without departing from the scope of the claims. Accordingly, the invention is to be defined not by the preceding illustrative description but instead by the scope of the following claims.

## Claims

1. An interventional device, comprising:
a sonoluminescent light module for placement inside a body, the light module comprising:
a housing (103);
an acoustic transducer (110) disposed inside the housing for generating a sound wave;
an acoustic conducting medium (105) disposed inside the housing and adjacent the acoustic transducer; and
a lens (109) disposed between the acoustic transducer (110) and the acoustic conducting medium (105), focusing the sound wave generated by the acoustic transducer (110) in the acoustic conducting medium (105) for producing light.

2. The interventional device of claim 1 wherein the acoustic transducer (110) comprises a piezoelectric material and a wave matching layer (107).

3. The interventional device of claim 2 wherein the piezoelectric material (113) comprises lead zirconate-titanate.

4. The interventional device of claim 1 wherein a distal end of the housing (103) is shaped to provide reflection and concentration of sound waves in the acoustic conducting medium (115).

5. The interventional device of claim 1 wherein the acoustic conducting medium (115) comprises water.

6. The interventional device of claim 1 wherein the acoustic conducting medium comprises a solid substance or target on which sonoluminescent effect can be observed.

7. The interventional device of claim 1 wherein the sonoluminescent light module is disposed near a distal end (117) of the interventional device.

8. The interventional device of claim 1 further comprising a pulse generator (112) in communication with the sonoluminescent light module through electrical conduits (114) positioned inside the interventional device.

9. The interventional device of claim 1 wherein the distal end of the interventional device performs as the housing.

10. The interventional device of claim I wherein at least a portion of the interventional device comprises an optically transparent material.

11. The interventional device of claim 1 wherein the position of the light module inside the interventional device is adjustable.

12. The interventional device of claim 1 wherein the interventional device has an optically transparent window comprising a material selected to transmit light having a predetermined wavelength.

13. The interventional device of claim 1 wherein the light produced comprises x-rays.

14. An interventional device, comprising:
a sonoluminescent light module for placement inside a body, the light module comprising:
a housing (103);
an acoustic transducer (110) disposed inside the housing for generating a sound wave;
an acoustic conducting medium (115) disposed inside the housing and adjacent the acoustic transducer; and
wherein a distal end of the housing is shaped to provide reflection and concentration of sound waves in the acoustic conducting medium for producing light.

15. The interventional device of claim 14 wherein the acoustic transducer (110) comprises a piezoelectric material and a wave matching layer (107).

16. The interventional device of claim 15 wherein the piezoelectric material (113) comprises lead zirconate-titanate.

17. The interventional device of claim 14 wherein the acoustic conducting medium comprises a solid substance or target on which sonoluminescent effect can be observed.

18. The interventional device of claim 14 wherein the sonoluminescent light module is disposed near a distal end (117) of the interventional device.

19. The interventional device of claim 14 further comprising a pulse generator (112) in communication with the sonoluminescent light module through electrical conduits positioned inside the interventional device.

20. The interventional device of claim 14 wherein the position of the light module inside the interventional device is adjustable.

21. The interventional device of claim 14 wherein the interventional device has an optically transparent window comprising a material selected to transmit light having a predetermined wavelength.

22. The interventional device of claim 14 wherein the light produced comprises x-rays.

## Patentansprüche

1. Eingriffsvorrichtung mit:
einem sonolumineszenten Lichtmodul zur Platzierung innerhalb eines Körpers, wobei das Lichtmodul umfasst:
ein Gehäuse (103);
einen akustischen Wandler (110), der innerhalb des Gehäuses zum Erzeugen einer Schallwelle angeordnet ist;
ein akustisches leitendes Medium (105), das innerhalb des Gehäuses und benachbart dem akustischen Wandler angeordnet ist; und
eine Linse (109), die zwischen dem akustischen Wandler (110) und dem akustischen leitenden Medium (105) angeordnet ist, die die von dem akustischen Wandler (110) in dem akustischen leitenden Medium (105) erzeugte Schallwelle zum Erzeugen von Licht fokussiert.

2. Eingriffsvorrichtung gemäß Anspruch 1, bei der der akustische Wandler (110) ein piezoelektrisches Material und eine Wellenanpassungsschicht (107) umfasst.

3. Eingriffsvorrichtung gemäß Anspruch 2, bei der das piezoelektrische Material (113) Blei-Zirkonat-Titanat umfasst.

4. Eingriffsvorrichtung gemäß Anspruch 1, bei der ein distales Ende des Gehäuses (103) geformt ist, um Reflexion und Konzentration von Schallwellen in dem akustischen leitenden Medium (115) bereitzustellen.

5. Eingriffsvorrichtung gemäß Anspruch 1, bei der das akustische leitende Medium (115) Wasser umfasst.

6. Eingriffsvorrichtung gemäß Anspruch 1, bei der das akustische leitende Medium eine feste Substanz oder Ziel umfasst, auf der eine sonolumineszente Wirkung beobachtet werden kann.

7. Eingriffsvorrichtung gemäß Anspruch 1, bei der das sonolumineszente Lichtmodul in der Nähe eines distalen Endes (117) der Eingriffsvorrichtung angeordnet ist.

8. Eingriffsvorrichtung gemäß Anspruch 1, ferner mit einem Impulsgenerator (112), der mit dem sonolumineszenten Lichtmodul durch innerhalb der Eingriffsvorrichtung positionierten elektrischen Leitungen (114) in Kommunikation ist.

9. Eingriffsvorrichtung gemäß Anspruch 1, bei der das distale Ende der Eingriffsvorrichtung als das Gehäuse arbeitet.

10. Eingriffsvorrichtung gemäß Anspruch 1, bei der mindestens ein Abschnitt der Eingriffsvorrichtung ein optisch transparentes Material umfasst.

11. Eingriffsvorrichtung gemäß Anspruch 1, bei der die Position des Lichtmoduls innerhalb der Eingriffsvorrichtung einstellbar ist.

12. Eingriffsvorrichtung gemäß Anspruch 1, bei der die Eingriffsvorrichtung ein optisch transparentes Fenster aufweist, das ein Material umfasst, das ausgewählt ist, um Licht mit einer vorbestimmten Wellenlänge durchzulassen.

13. Eingriffsvorrichtung gemäß Anspruch 1, bei der das erzeugte Licht Röntgenstrahlen umfasst.

14. Eingriffsvorrichtung mit:
einem sonolumineszenten Lichtmodul zur Platzierung innerhalb eines Körpers, wobei das Lichtmodul umfasst:
ein Gehäuse (103);
einen akustischen Wandler (110), der innerhalb des Gehäuses zum Erzeugen einer Schallwelle angeordnet ist;
ein akustisches leitendes Medium (115), das innerhalb des Gehäuse und benachbart dem akustischen Wandler angeordnet ist; und
wobei ein distales Ende des Gehäuses geformt ist, um eine Reflexion und Konzentration der Schallwellen in dem akustischen leitenden Medium zum Erzeugen von Licht bereitzustellen.

15. Eingriffsvorrichtung gemäß Anspruch 14, bei der der akustische Wandler (110) ein piezoelektrisches Material und eine Wellenanpassungsschicht (107) umfasst.

16. Eingriffsvorrichtung gemäß Anspruch 15, bei der das piezoelektrische Material (113) Blei-Zirkonat-Titanat umfasst.

17. Eingriffsvorrichtung gemäß Anspruch 14, bei der das akustische leitende Medium eine feste Substanz oder Ziel umfasst, auf der ein sonolumineszente Wirkung beobachtet werden kann.

18. Eingriffsvorrichtung gemäß Anspruch 14, bei der das sonolumineszente Lichtmodul in der Nähe eines distalen Endes (117) der Eingriffsvorrichtung angeordnet ist.

19. Eingriffsvorrichtung gemäß Anspruch 14, ferner mit einem Impulsgenerator (112), der mit dem sonolumineszenten Lichtmodul durch innerhalb der Eingriffsvorrichtung positionierten elektrischen Leitungen in Kommunikation ist.

20. Eingriffsvorrichtung gemäß Anspruch 14, bei der die Position des Lichtmoduls innerhalb der Eingriffsvorrichtung einstellbar ist.

21. Eingriffsvorrichtung gemäß Anspruch 14, bei der die Eingriffsvorrichtung ein optisch transparentes Fenster aufweist, das ein Material umfasst, das ausgewählt ist, um Licht mit einer vorbestimmten Wellenlänge durchzulassen.

22. Eingriffsvorrichtung gemäß Anspruch 14, bei der das erzeugte Licht Röntgenstrahlen umfasst.

## Revendications

1. Dispositif d'intervention, comprenant :
un module lumineux sonoluminescent pour la mise en place à l'intérieur d'un corps, le module lumineux comprenant :
une enceinte (103) ;
un transducteur acoustique (110) disposé à l'intérieur de l'enceinte pour générer une onde acoustique ;
un conducteur électrique acoustique (105) disposé à l'intérieur de l'enceinte et adjacent au transducteur acoustique ; et
une lentille (109) disposée entre le transducteur acoustique (110) et le conducteur électrique acoustique (105), adaptée pour concentrer l'onde acoustique générée par le transducteur acoustique (110) dans le conducteur électrique acoustique (105) afin de produire de la lumière.

2. Dispositif d'intervention selon la revendication 1 dans lequel le transducteur acoustique (110) comprend un matériau piézoélectrique et une couche correspondant à une onde (107).

3. Dispositif d'intervention selon la revendication 2 dans lequel le matériau piézoélectrique (113) comprend du zirconate-titanate de plomb.

4. Dispositif d'intervention selon la revendication 1 dans lequel une extrémité distale de l'enceinte (103) est formée de manière à permettre la réflexion et la concentration d'ondes acoustiques dans le conducteur électrique acoustique (115).

5. Dispositif d'intervention selon la revendication 1 dans lequel le conducteur électrique acoustique (115) comprend de l'eau.

6. Dispositif d'intervention selon la revendication 1 dans lequel le conducteur électrique acoustique comprend une substance solide ou une cible sur laquelle l'effet sonoluminescent peut être observé.

7. Dispositif d'intervention selon la revendication 1 dans lequel le module lumineux sonoluminescent est disposé à proximité d'une extrémité distale (117) du dispositif d'intervention.

8. Dispositif d'intervention selon la revendication 1 comprenant en outre un générateur d'impulsion (112) qui est en communication avec le module lumineux sonoluminescent à travers des conduites électrique (114) logées à l'intérieur du dispositif d'intervention.

9. Dispositif d'intervention selon la revendication 1 dans lequel l'extrémité distale du dispositif d'intervention agit en tant que l'enceinte.

10. Dispositif d'intervention selon la revendication 1 dans lequel au moins une partie du dispositif d'intervention comprend un matériau optiquement transparent.

11. Dispositif d'intervention selon la revendication 1 dans lequel la position du module lumineux à l'intérieur du dispositif d'intervention est réglable.

12. Dispositif d'intervention selon la revendication 1 dans lequel le dispositif d'intervention comporte une fenêtre optiquement transparente comprenant un matériau choisi pour transmettre une lumière ayant une longueur d'onde prédéterminée.

13. Dispositif d'intervention selon la revendication 1 dans lequel la lumière produite comprend des rayons X.

14. Dispositif d'intervention comprenant :
un module lumineux sonoluminescent pour la mise en place à l'intérieur d'un corps, le module lumineux comprenant :
une enceinte (103) ;
un transducteur acoustique (110) disposé à l'intérieur de l'enceinte pour générer une onde acoustique ;
un conducteur électrique acoustique (115) disposé à l'intérieur de l'enceinte et adjacent au transducteur acoustique ; et
dans lequel une extrémité distale de l'enceinte est formée de manière à permettre la réflexion et la concentration d'ondes acoustiques dans le conducteur électrique acoustique afin de produire de la lumière.

15. Dispositif d'intervention selon la revendication 14 dans lequel le transducteur acoustique (110) comprend un matériau piézoélectrique et une couche correspondant à une onde (107).

16. Dispositif d'intervention selon la revendication 15 dans lequel le matériau piézoélectrique (113) comprend du zirconate-titanate de plomb.

17. Dispositif d'intervention selon la revendication 14 dans lequel le conducteur électrique acoustique comprend une substance solide ou une cible sur laquelle l'effet sonoluminescent peut être observé.

18. Dispositif d'intervention selon la revendication 14 dans lequel le module lumineux sonoluminescent est disposé à proximité d'une extrémité distale (117) du dispositif d'intervention.

19. Dispositif d'intervention selon la revendication 14 comprenant en outre un générateur d'impulsion qui est (112) en communication avec le module lumineux sonoluminescent à travers des conduites électriques logées à l'intérieur du dispositif d'intervention.

20. Dispositif d'intervention selon la revendication 14 dans lequel la position du module lumineux à l'intérieur du dispositif d'intervention est réglable.

21. Dispositif d'intervention selon la revendication 14 dans lequel le dispositif d'intervention comporte une fenêtre optiquement transparente comprenant un matériau choisi pour transmettre une lumière ayant une longueur d'onde prédéterminée.

22. Dispositif d'intervention selon la revendication 14 dans lequel la lumière produite comprend des rayons X.
